# EUROPEAN PATENT SPECIFICATION

(11) **EP 2 574 194 B1**
(45) Date of publication and mention of the grant of the patent: **11.10.2017**
(21) Application number: 11784375.5
(22) Date of filing: 23.05.2011
(51) Int. Cl.: C07D 513/16, C07D 513/14, A61K 31/429, A61P 19/10, A61K 45/06

(54) **MACROCYCLIC COMPOUNDS FOR USE IN THE TREATMENT OF OSTEOPOROSIS**
MAKROCYCLISCHE VERBINDUNGEN ZUR VERWENDUNG IN DER BEHANDLUNG VON OSTEOPOROSE
COMPOSÉS MACROCYCLIQUES POUR LEUR UTILISATION DANS LE TRAITEMENT DE L'OSTEOPOROSE

(30) Priority: 21.05.2010 US 347109 P; 25.10.2010 US 406413 P
(43) Date of publication of application: 03.04.2013
(73) Proprietor: University of Florida Research Foundation, Inc., Gainesville, Florida 32611-5500 (US); Duke University, Durham, NC 27705 (US); Korea Research Institute of Chemical Technology, Yuseong-gu, Daejeon 305-343 (KR)
(72) Inventor: LUESCH, Hendrik, Gainesville, FL 32607 (US); HONG, Jiyong, Durham, NC 27708 (US); KIM, Seong, Hwan, Daejeon 305-600 (KR)
(74) Representative: Plougmann Vingtoft a/s
(86) International application number: PCT/US2011/037545
(87) International publication number: WO 2011/146918

(56) References cited:
- WO-A1-2009/032352
- WO-A1-2009/105284
- CHO HYUN HWA ET AL: "Induction of osteogenic differentiation of human mesenchymal stem cells by histone deacetylase inhibitors", JOURNAL OF CELLULAR BIOCHEMISTRY, WILEY-LISS INC, US, vol. 96, no. 3, 15 October 2005 (2005-10-15), pages 533-542, XP002533700, ISSN: 0730-2312, DOI: 10.1002/JCB.20544
- SCHROEDER TANIA M ET AL: "Histone deacetylase inhibitors promote osteoblast maturation", JOURNAL OF BONE AND MINERAL RESEARCH, BLACKWELL SCIENCE, INC, vol. 20, no. 12, 1 December 2005 (2005-12-01), pages 2254-2263, XP002490859, ISSN: 0884-0431, DOI: 10.1359/JBMR.050813
- MASUYA KEIICHI ET AL: "Small molecules for bone diseases", EXPERT OPINION ON THERAPEUTIC PATENTS, INFORMA HEALTHCARE, GB, vol. 20, no. 4, 1 April 2010 (2010-04-01), pages 563-582, XP009148302, ISSN: 1354-3776
- SU-UI LEE ET AL: "In Vitro and In Vivo Osteogenic Activity of Largazole", ACS MEDICINAL CHEMISTRY LETTERS, vol. 2, no. 3, 10 March 2011 (2011-03-10), pages 248-251, XP055084782, ISSN: 1948-5875, DOI: 10.1021/ml1002794
- GHOSH, A. K. ET AL.: 'Enantioselective Total Synthesis of (+)-Largazole, a Potent Inhibitor of Histone Deacetylase' ORGANIC LETTERS vol. 10, no. 17, 2008, pages 3907 - 3909, XP055019296
- SEISER, T. ET AL.: 'Synthesis and Biological Activity of Largazole and Derivatives' ANGEWANDTE CHEMIE. INTERNATIONAL EDITION vol. 47, no. 34, 2008, pages 6483 - 6485, XP055019289

## Description

### BACKGROUND

The identification of new pharmacophores is of paramount biomedical importance and natural products have recently been regaining attention for this endeavor.¹ This renaissance is closely tied to the successful exploitation of the marine environment which harbors unmatched biodiversity that is presumably concomitant with chemical diversity.² In particular, marine cyanobacteria are prolific producers of bioactive secondary metabolites,³ many of which are modified peptides or peptide-polyketide hybrids with promising antitumor activities, such as dolastatin 10,⁴ curacin A,⁵ and apratoxin A.⁶ As a result of ongoing investigations to identify new drug leads from cyanobacteria in Florida, we report here the structure determination and preliminary biological characterization of a marine cyanobacterial metabolite with novel chemical scaffold and nanomolar antiproliferative activity. These findings provide new alternatives to address unmet needs in the treatment of proliferation diseases and disorders. The compounds herein are also now found to mediate bone disease/disorders processes (e.g., osteoclastogenesis, osteoblastogenesis) and as such are useful for treating diseases, disorders, or symptoms thereof mediated by such processes. These findings provide new alternatives to address unmet needs in the treatment of bone diseases and disorders.

### BRIEF SUMMARY OF THE INVENTION

The invention is directed towards pharmaceutical compositions comprising certain macrocyclic compounds of formula (I) and an antiosteoporotic agent and the macrocyclic compounds of formula (I) for use in treating osteoporosis.

In one embodiment, the invention provides a pharmaceutical composition comprising a compound according to Formula I: 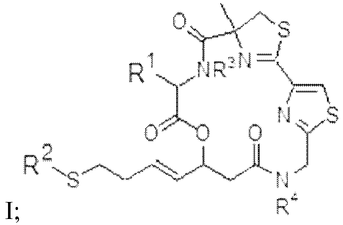 wherein:
each R is independently H or alkyl;
each R¹ is independently H, or alkyl;
each R² is independently H, alkyl, or C(O)R;
each R³ is independently H, alkyl, C(O)OR, or C(O)NRR;
each R⁴ is independently H, alkyl, C(O)OR, or C(O)NRR;
and pharmaceutically acceptable salts, solvates, or hydrates thereof, an anti-osteoporotic agent and a pharmaceutically acceptable carrier.

The compound of formula I may be a compound of formula Ia (and pharmaceutically acceptable salts, solvates, or hydrates thereof), where R, R¹, R², R³, and R⁴ are as defined in formula I:

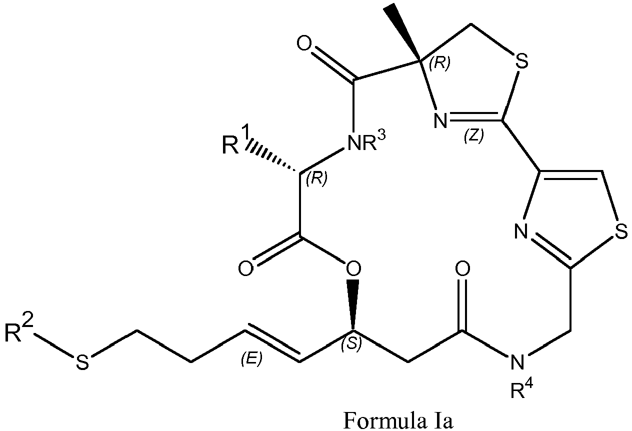

Other embodiments include a compound of any of the formulae herein, wherein R³ and R⁴ are H; wherein R¹ is isopropyl; wherein R² is alkyl; wherein R² is alkylC(O)-; wherein R² is H; wherein the compound is any of Compounds 1-8 in Table A; or wherein the compound is largazole.

In certain instances, the compounds in the compositions of the invention are selected from the following of Formula (I) (including formula Ia) having the structure:

**Table A**

| Cmpd No. | R¹ | R² | R³ | R⁴ |
|---|---|---|---|---|
| 1 | isopropyl | n-heptylC(O)- | H | H |
| 2 | isopropyl | n-heptylC(O)- | H | Me |
| 3 | isopropyl | Me | H | H |
| 4 | isopropyl | n-heptylC(O)- | H | methylC(O)- |
| 5 | isopentyl | n-heptylC(O)- | H | H |
| 6 | Ethyl | n-heptylC(O)- | Me | Me |
| 7 | isopropyl | CH₃C(O)- | H | H |
| 8 | isopropyl | H | H | H |

In another aspect, the invention provides a compound of formula I as defined above for use in treating osteoporosis.

The invention enables a method of treating a bone disease, disorder, or symptom thereof in a subject, comprising administering to the subject a compound of any of the formulae herein (e.g., formula I, formula Ia). In another aspect, the compound is administered in an amount and under conditions sufficient to ameliorate the bone disease, disorder, or symptom thereof in a subject.

The invention also enables a method of modulating osteoclastogenesis activity in a subject, comprising contacting the subject with a compound of any of the formulae herein (e.g., formula I, formula Ia), in an amount and under conditions sufficient to modulate osteoclastogenesis activity. In another aspect, the modulation is inhibition.

The invention also enables a method of modulating osteoblastogenesis activity in a subject, comprising contacting the subject with a compound of formula I, in an amount and under conditions sufficient to modulate osteoblastogenesis activity.

Further, the invention enables a method of treating a subject suffering from or susceptible to a bone related disorder or disease, comprising administering to the subject an effective amount of a compound or pharmaceutical composition of formula I.

The invention further enables a method of treating a subject suffering from or susceptible to a osteoclasto-related activity related disorder or disease, wherein the subject has been identified as in need of treatment for a osteoclasto-related disorder or disease, comprising administering to said subject in need thereof, an effective amount of a compound or pharmaceutical composition of formula I, such that said subject is treated for said disorder.

The invention further enables a method of treating a subject suffering from or susceptible to a osteoblasto-related disorder or disease, wherein the subject has been identified as in need of treatment for a osteoblasto related disorder or disease, comprising administering to said subject in need thereof, an effective amount of a compound or pharmaceutical composition of formula I, such that cell proliferation in said subject is modulated (e.g., down regulated).

In a specific aspect, the invention enables a method of treating osteoporosis, bone formation, resorption, bone regeneration, bone tissue engineering, fracture, periodontal disease, bone growth disorder.

Thus, certain compounds described_herein are useful to inhibit osteoclastogenesis. In another aspect, certain herein are useful to promote osteoblastogenesis. In another aspect, certain compounds herein are useful for their dual activity (e.g., anabolic activity and resorptive activity).

Certain compounds described herein are useful for their dual action to stimulate bone formation and inhibit bone resorption.

The invention further enables a method of treating diseases, disorders, or symptoms in a subject in need thereof comprising administering to said subject, an effective amount of a compound delineated herein (e.g., Formula I), and pharmaceutically acceptable salts thereof. Such methods are useful for treating bone related disorders described herein.

### BRIEF DESCRIPTION OF THE DRAWINGS

The present invention is further described below with reference to the following non-limiting examples and with reference to the following figures, in which:

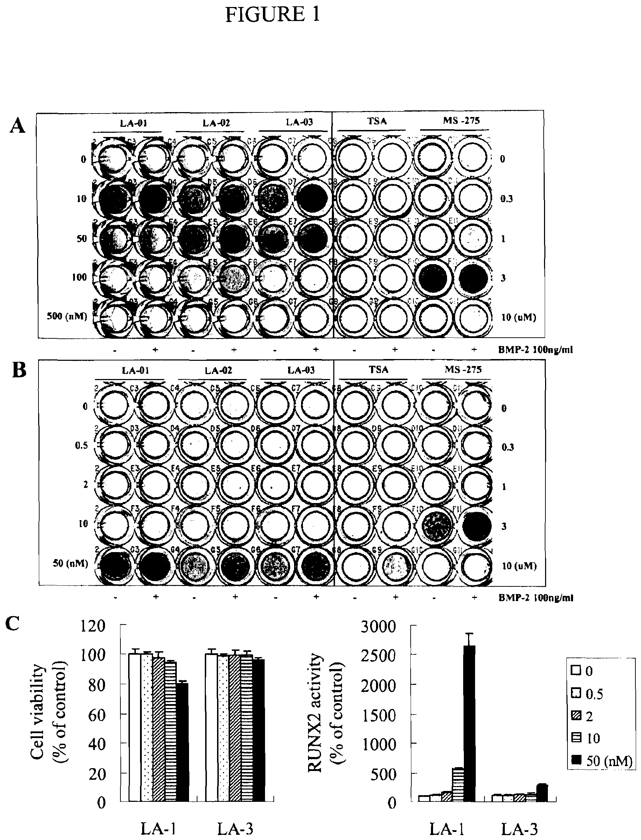

FIG 1. depicts activity of compound 1 (LA-1) and compound 8 (LA-3) on osteoblast differentiation. Figure 1: (A) The effects of test compounds (up to 500 nM) on osteoblast differentiation were evaluated by ALP staining. (B) The effect of test compounds (up to 50 nM) on osteoblast differentiation were evaluated by ALP staining. MS-275 was used as a positive control. The effects of test compounds on cell viability (C) and RUNX2 activity (D) were evaluated by CCK-8 assay and 6×OSE2-luciferase activity assay, respectively.

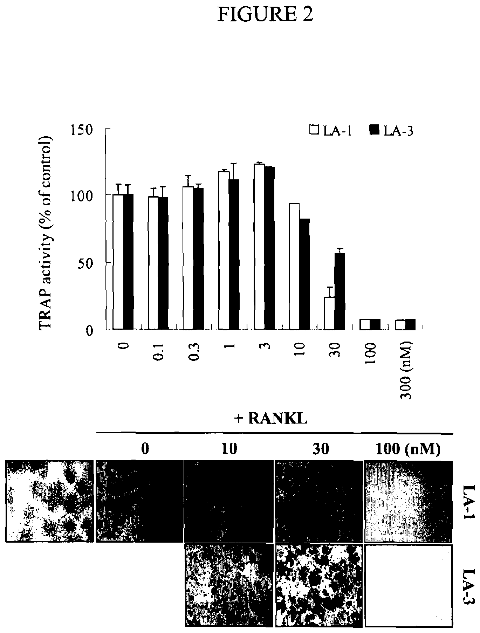

FIG 2 depicts activity of compound 1 (LA-1) and compound 8 (LA-3) on osteoclastogenesis. Figure 2: (A) Effects of test compounds on the RANKL-induced TRAP activity. (B) Effects of test compounds on the RANKL-induced formation of TRAP-positive multinucleated osteoclasts. Effects of test compounds on osteoclast differentiation was evaluated in RAW264.7 cells. RAW264.7 cells were plated in 96-well plates at the density 1 x 10³ and then serially diluted LA compounds were treated and incubated until multinucleated osteoclasts were observed under a microscope.

### DETAILED DESCRIPTION

### Definitions

In order that the invention may be more readily understood, certain terms are first defined here for convenience.

As used herein, the term "treating" a disorder encompasses preventing, ameliorating, mitigating and/or managing the disorder and/or conditions that may cause the disorder. The terms "treating" and "treatment" refer to a method of alleviating or abating a disease and/or its attendant symptoms. In accordance with the present invention "treating" includes preventing, blocking, inhibiting, attenuating, protecting against, modulating, reversing the effects of and reducing the occurrence of e.g., the harmful effects of a disorder.

As used herein, "inhibiting" encompasses preventing, reducing and halting progression.

The term "modulate" refers to increases or decreases in the activity of a cell in response to exposure to a compound of the invention.

The terms "isolated," "purified," or "biologically pure" refer to material that is substantially or essentially free from components that normally accompany it as found in its native state. Purity and homogeneity are typically determined using analytical chemistry techniques such as polyacrylamide gel electrophoresis or high performance liquid chromatography. Particularly, in embodiments the compound is at least 85% pure, more preferably at least 90% pure, more preferably at least 95% pure, and most preferably at least 99% pure.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

A "peptide" is a sequence of at least two amino acids. Peptides can consist of short as well as long amino acid sequences, including proteins.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., an a carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

The term "protein" refers to series of amino acid residues connected one to the other by peptide bonds between the alpha-amino and carboxy groups of adjacent residues.

Amino acids may be referred to herein by either their commonly known three letter symbols or by the one-letter symbols recommended by the IUPAC-IUB Biochemical Nomenclature Commission.

As to amino acid sequences, one of skill will recognize that individual substitutions, deletions or additions to a peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art.

Macromolecular structures such as polypeptide structures can be described in terms of various levels of organization. For a general discussion of this organization, see, e.g., Alberts et al., Molecular Biology of the Cell (3rd ed., 1994) and Cantor and Schimmel, Biophysical Chemistry Part I. The Conformation of Biological Macromolecules (1980). "Primary structure" refers to the amino acid sequence of a particular peptide. "Secondary structure" refers to locally ordered, three dimensional structures within a polypeptide. These structures are commonly known as domains. Domains are portions of a polypeptide that form a compact unit of the polypeptide and are typically 50 to 350 amino acids long. Typical domains are made up of sections of lesser organization such as stretches of β-sheet and α-helices. "Tertiary structure" refers to the complete three dimensional structure of a polypeptide monomer. "Quaternary structure" refers to the three dimensional structure formed by the noncovalent association of independent tertiary units. Anisotropic terms are also known as energy terms.

The term "administration" or "administering" includes routes of introducing the compound(s) to a subject to perform their intended function. Examples of routes of administration which can be used include injection (subcutaneous, intravenous, parenterally, intraperitoneally, intrathecal), topical, oral, inhalation, rectal and transdermal.

The term "effective amount" includes an amount effective, at dosages and for periods of time necessary, to achieve the desired result. An effective amount of compound may vary according to factors such as the disease state, age, and weight of the subject, and the ability of the compound to elicit a desired response in the subject. Dosage regimens may be adjusted to provide the optimum therapeutic response. An effective amount is also one in which any toxic or detrimental effects (*e.g.,* side effects) of the elastase inhibitor compound are outweighed by the therapeutically beneficial effects.

The phrases "systemic administration," "administered systemically", "peripheral administration" and "administered peripherally" as used herein mean the administration of a compound(s), drug or other material, such that it enters the patient's system and, thus, is subject to metabolism and other like processes.

The term "therapeutically effective amount" refers to that amount of the compound being administered sufficient to prevent development of or alleviate to some extent one or more of the symptoms of the condition or disorder being treated.

A therapeutically effective amount of compound (*i.e.,* an effective dosage) may range from about 0.005 µg/kg to about 200 mg/kg, preferably about 0.1 mg/kg to about 200 mg/kg, more preferably about 10 mg/kg to about 100 mg/kg of body weight. In other embodiments, the therapeutically effect amount may range from about 1.0 pM to about 500nM. The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of a compound can include a single treatment or, preferably, can include a series of treatments. In one example, a subject is treated with a compound in the range of between about 0.005 µg/kg to about 200 mg/kg of body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage of a compound used for treatment may increase or decrease over the course of a particular treatment.

The term "chiral" refers to molecules which have the property of non-superimposability of the mirror image partner, while the term "achiral" refers to molecules which are superimposable on their mirror image partner.

The term "diastereomers" refers to stereoisomers with two or more centers of dissymmetry and whose molecules are not mirror images of one another.

The term "enantiomers" refers to two stereoisomers of a compound which are non-superimposable mirror images of one another. An equimolar mixture of two enantiomers is called a "racemic mixture" or a "racemate."

The term "isomers" or "stereoisomers" refers to compounds which have identical chemical constitution, but differ with regard to the arrangement of the atoms or groups in space.

The term "subject" refers to animals such as mammals, including, but not limited to, primates (e.g., humans), cows, sheep, goats, horses, dogs, cats, rabbits, rats, mice and the like. In certain embodiments, the subject is a human.

As used herein, the term "alkyl" refers to a straight-chained or branched hydrocarbon group containing 1 to 12 carbon atoms. The term "lower alkyl" refers to a C1-C6 alkyl chain. Examples of alkyl groups include methyl, ethyl, n-propyl, isopropyl, *tert*-butyl, and n-pentyl.

Acids and bases useful in the methods herein are known in the art. Acid catalysts are any acidic chemical, which can be inorganic (e.g., hydrochloric, sulfuric, nitric acids, aluminum trichloride) or organic (e.g., camphorsulfonic acid, p-toluenesulfonic acid, acetic acid, ytterbium triflate) in nature. Acids are useful in either catalytic or stoichiometric amounts to facilitate chemical reactions. Bases are any basic chemical, which can be inorganic (e.g., sodium bicarbonate, potassium hydroxide) or organic (e.g., triethylamine, pyridine) in nature. Bases are useful in either catalytic or stoichiometric amounts to facilitate chemical reactions.

Alkylating agents are any reagent that is capable of effecting the alkylation of the functional group at issue (e.g., oxygen atom of an alcohol, nitrogen atom of an amino group). Alkylating agents are known in the art, including in the references cited herein, and include alkyl halides (e.g., methyl iodide, benzyl bromide or chloride), alkyl sulfates (e.g., methyl sulfate), or other alkyl group-leaving group combinations known in the art. Leaving groups are any stable species that can detach from a molecule during a reaction (e.g., elimination reaction, substitution reaction) and are known in the art, including in the references cited herein, and include halides (e.g., I-, Cl-, Br-, F-), hydroxy, alkoxy (e.g., -OMe, -O-t-Bu), acyloxy anions (e.g., - OAc, -OC(O)CF₃), sulfonates (e.g., mesyl, tosyl), acetamides (e.g., -NHC(O)Me), carbamates (e.g., N(Me)C(O)Ot-Bu), phosphonates (e.g., -OP(O)(OEt)₂), water or alcohols (protic conditions), and the like.

### Compounds of the Invention

Compounds of the invention can be made by means known in the art of organic synthesis. Methods for optimizing reaction conditions, if necessary minimizing competing by-products, are known in the art. Reaction optimization and scale-up may advantageously utilize high-speed parallel synthesis equipment and computer-controlled microreactors (e.g. Design And Optimization in Organic Synthesis, 2nd Edition, Carlson R, Ed, 2005; Elsevier Science Ltd.; Jähnisch, K et al, Angew. Chem. Int. Ed. Engl. 2004 43: 406; and references therein). Additional reaction schemes and protocols may be determined by the skilled artesian by use of commercially available structure-searchable database software, for instance, SciFinder® (CAS division of the American Chemical Society) and CrossFire Beilstein® (Elsevier MDL), or by appropriate keyword searching using an internet search engine such as Google® or keyword databases such as the US Patent and Trademark Office text database.

The compounds herein may also contain linkages (e.g., carbon-carbon bonds) wherein bond rotation is restricted about that particular linkage, e.g. restriction resulting from the presence of a ring or double bond. Accordingly, all *cis*/*trans* and E/Z isomers are expressly included in the present invention. The compounds herein may also be represented in multiple tautomeric forms, in such instances, the invention expressly includes all tautomeric forms of the compounds described herein, even though only a single tautomeric form may be represented. All such isomeric forms of such compounds herein are expressly included in the present invention. All crystal forms and polymorphs of the compounds described herein are expressly included in the present invention. Also embodied are extracts and fractions comprising compounds of the invention. The term isomers is intended to include diastereoisomers, enantiomers, regioisomers, structural isomers, rotational isomers, tautomers, and the like. For compounds which contain one or more stereogenic centers, e.g., chiral compounds, the methods of the invention may be carried out with an enantiomerically enriched compound, a racemate, or a mixture of diastereomers.

Preferred enantiomerically enriched compounds have an enantiomeric excess of 50% or more, more preferably the compound has an enantiomeric excess of 60%, 70%, 80%, 90%, 95%, 98%, or 99% or more. In preferred embodiments, only one enantiomer or diastereomer of a chiral compound of the invention is administered to cells or a subject.

### Methods of Treatment

The invention is directed towards pharmaceutical compositions comprising certain macrocyclic compounds, and such compounds for use in treating osteoporosis.

In other aspects, the invention enables a method of treating a subject suffering from or susceptible to bone disorder or disease, wherein the subject has been identified as in need of treatment for a bone disorder or disease, comprising administering to said subject in need thereof, an effective amount of a compound or pharmaceutical composition of formula I, such that said subject is treated for said disorder. Identifying a subject in need of such treatment can be in the judgment of a subject or a health care professional and can be subjective (e.g. opinion) or objective (e.g. measurable by a test or diagnostic method).

Further, the invention enables a method of modulating the osteoclasto activity in a subject, comprising contacting the subject with a compound of formula I, in an amount and under conditions sufficient to modulate osteoclasto activity.

The modulation may be inhibition.

Further, the invention enables a method of treating a subject suffering from or susceptible to a osteoblasto related disorder or disease, comprising administering to the subject an effective amount of a compound or pharmaceutical composition of formula I.

Further, the invention enables a method of treating a subject suffering from or susceptible to a osteoclasto-mediated disorder or disease, wherein the subject has been identified as in need of treatment for a osteoclasto-mediated disorder or disease, comprising administering to said subject in need thereof, an effective amount of a compound or pharmaceutical composition of formula I, such that said subject is treated for said disorder.

Further, the invention enables a method of treating a subject suffering from or susceptible to a osteoblasto-mediated disorder or disease, wherein the subject has been identified as in need of treatment for a osteoblasto-mediated disorder or disease, comprising administering to said subject in need thereof, an effective amount of a compound or pharmaceutical composition of formula I, such that said subject is treated for said disorder.

In certain embodiments, the invention provides a composition or a compound for use as described above, wherein the compound of formula I is largazole.

In certain embodiments, the invention enables a method of treating a disorder, wherein the disorder is osteoporosis, bone tissue engineering, bone tissue regeneration, or other bone disorder. The compounds and methods herein are useful in bone formation, bone repair and bone regeneration in a subject.

In certain embodiments, the subject is a mammal, preferably a primate or human.

In another embodiment, the invention enables a method as described above, wherein the effective amount of the compound of formula I ranges from about 0.005 µg/kg to about 200 mg/kg. In certain embodiments, the effective amount of the compound of formula I ranges from about 0.1 mg/kg to about 200 mg/kg. In a further embodiment, the effective amount of compound of formula I ranges from about 10 mg/kg to 100 mg/kg.

The invention further enables a method as described above wherein the effective amount of the compound of formula I ranges from about 1.0 pM to about 500 nM. In certain embodiments, the effective amount ranges from about 10.0 pM to about 1000 pM. In another embodiment, the effective amount ranges from about 1.0 nM to about 10 nM.

The invention further enables a method as described above, wherein the compound of formula I is administered intravenously, intramuscularly, subcutaneously, intracerebroventricularly, orally or topically.

The invention also enables a method as described above, wherein the compound of formula I is administered alone or in combination with one or more other therapeutics. The additional therapeutic agent-may be an anti-osteoporotic agent, agent for treating bone resorption-related disease, fracture healing agent, bone forming agent.

### Pharmaceutical Compositions

In one aspect, the invention provides a pharmaceutical composition comprising the compound of formula I and a pharmaceutically acceptable carrier.

In one embodiment, the invention provides a pharmaceutical composition wherein the compound of formula I is largazole, and a pharmaceutically acceptable carrier.

The pharmaceutical composition may further comprise an additional therapeutic agent such as an anti-osteoporotic agent.

In one aspect, the invention provides a kit comprising an effective amount of a compound of formula I, in unit dosage form, for use in treating osteoporosis

The term "pharmaceutically acceptable salts" or "pharmaceutically acceptable carrier" is meant to include salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, e.g., Berge et al., Journal of Pharmaceutical Science 66:1-19 (1977)). Certain specific compounds of the present invention contain both basic and acidic functionalities that allow the compounds to be converted into either base or acid addition salts. Other pharmaceutically acceptable carriers known to those of skill in the art are suitable for the present invention.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

In addition to salt forms, the present invention provides compounds which are in a prodrug form. Prodrugs of the compounds described herein are those compounds that readily undergo chemical changes under physiological conditions to provide the compounds of the present invention. Additionally, prodrugs can be converted to the compounds of the present invention by chemical or biochemical methods in an ex vivo environment. For example, prodrugs can be slowly converted to the compounds of the present invention when placed in a transdermal patch reservoir with a suitable enzyme or chemical reagent.

Certain compounds of the present invention can exist in unsolvated forms as well as solvated forms, including hydrated forms. In general, the solvated forms are equivalent to unsolvated forms and are intended to be encompassed within the scope of the present invention. Certain compounds of the present invention may exist in multiple crystalline or amorphous forms. In general, all physical forms are equivalent for the uses contemplated by the present invention and are intended to be within the scope of the present invention.

The invention also provides a pharmaceutical composition, comprising an effective amount a compound described herein and a pharmaceutically acceptable carrier. In an embodiment, compound is administered to the subject using a pharmaceutically-acceptable formulation, e.g., a pharmaceutically-acceptable formulation that provides sustained delivery of the compound to a subject for at least 12 hours, 24 hours, 36 hours, 48 hours, one week, two weeks, three weeks, or four weeks after the pharmaceutically-acceptable formulation is administered to the subject.

Actual dosage levels and time course of administration of the active ingredients in the pharmaceutical compositions of this invention may be varied so as to obtain an amount of the active ingredient which is effective to achieve the desired therapeutic response for a particular patient, composition, and mode of administration, without being toxic (or unacceptably toxic) to the patient.

In use, at least one compound according to the present invention is administered in a pharmaceutically effective amount to a subject in need thereof in a pharmaceutical carrier by intravenous, intramuscular, subcutaneous, or intracerebroventricular injection or by oral administration or topical application. In accordance with the present invention, a compound of the invention may be administered alone or in conjunction with a second, different therapeutic. By "in conjunction with" is meant together, substantially simultaneously or sequentially. In one embodiment, a compound of the invention is administered acutely. The compound of the invention may therefore be administered for a short course of treatment, such as for about 1 day to about 1 week. In another embodiment, the compound of the invention may be administered over a longer period of time to ameliorate chronic disorders, such as, for example, for about one week to several months depending upon the condition to be treated.

By "pharmaceutically effective amount" as used herein is meant an amount of a compound of the invention, high enough to significantly positively modify the condition to be treated but low enough to avoid serious side effects (at a reasonable benefit/risk ratio), within the scope of sound medical judgment. A pharmaceutically effective amount of a compound of the invention will vary with the particular goal to be achieved, the age and physical condition of the patient being treated, the severity of the underlying disease, the duration of treatment, the nature of concurrent therapy and the specific organozinc compound employed. For example, a therapeutically effective amount of a compound of the invention administered to a child or a neonate will be reduced proportionately in accordance with sound medical judgment. The effective amount of a compound of the invention will thus be the minimum amount which will provide the desired effect.

A decided practical advantage of the present invention is that the compound may be administered in a convenient manner such as by intravenous, intramuscular, subcutaneous, oral or intra-cerebroventricular injection routes or by topical application, such as in creams or gels. Depending on the route of administration, the active ingredients which comprise a compound of the invention may be required to be coated in a material to protect the compound from the action of enzymes, acids and other natural conditions which may inactivate the compound. In order to administer a compound of the invention by other than parenteral administration, the compound can be coated by, or administered with, a material to prevent inactivation.

The compound may be administered parenterally or intraperitoneally. Dispersions can also be prepared, for example, in glycerol, liquid polyethylene glycols, and mixtures thereof, and in oils.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy syringability exists. It must be stable under the conditions of manufacture and storage. The carrier can be a solvent or dispersion medium containing, for example, water, DMSO, ethanol, polyol (for example, glycerol, propylene glycol, liquid polyethylene glycol, and the like), suitable mixtures thereof and vegetable oils. The proper fluidity can be maintained, for example, by the use of a coating such as lecithin, by the maintenance of the required particle size in the case of dispersion. In many cases it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the compound of the invention in the required amount in the appropriate solvent with various of the other ingredients enumerated above, as required, followed by filtered sterilization. Generally, dispersions are prepared by incorporating the various sterilized compounds into a sterile vehicle which contains the basic dispersion medium and the required other ingredients from those enumerated above. In the case of sterile powders for the preparation of sterile injectable solutions, the preferred methods of preparation are vacuum-drying and the freeze-drying technique which yields a powder of the active ingredient plus any additional desired ingredient from previously sterile-filtered solution thereof.

For oral therapeutic administration, the compound may be incorporated with excipients and used in the form of ingestible tablets, buccal tablets, troches, capsules, elixirs, suspensions, syrups, wafers, and the like. Compositions or preparations according to the present invention are prepared so that an oral dosage unit form contains compound concentration sufficient to treat a disorder in a subject.

Some examples of substances which can serve as pharmaceutical carriers are sugars, such as lactose, glucose and sucrose; starches such as corn starch and potato starch; cellulose and its derivatives such as sodium carboxymethycellulose, ethylcellulose and cellulose acetates; powdered tragancanth; malt; gelatin; talc; stearic acids; magnesium stearate; calcium sulfate; vegetable oils, such as peanut oils, cotton seed oil, sesame oil, olive oil, corn oil and oil of theobroma; polyols such as propylene glycol, glycerine, sorbitol, mannitol, and polyethylene glycol; agar; alginic acids; pyrogen-free water; isotonic saline; and phosphate buffer solution; skim milk powder; as well as other non-toxic compatible substances used in pharmaceutical formulations such as Vitamin C, estrogen and echinacea, for example. Wetting agents and lubricants such as sodium lauryl sulfate, as well as coloring agents, flavoring agents, lubricants, excipients, tableting agents, stabilizers, anti-oxidants and preservatives, can also be present.

The recitation of a listing of chemical groups in any definition of a variable herein includes definitions of that variable as any single group or combination of listed groups. The recitation of an embodiment for a variable herein includes that embodiment as any single embodiment or in combination with any other embodiments or portions thereof.

### Examples

The present invention will now be demonstrated using specific examples that are not to be construed as limiting.

### General Experimental Procedures.

¹H and ¹³C NMR data were acquired on a Bruker Avance 600 MHz spectrometer with a 5-mm probe operating at 600 and 150 MHz, respectively. 2D NMR data were recorded on a Bruker Avance II 600 MHz equipped with a 1-mm triple resonance high-temperature superconducting cryogenic probe using residual solvent signals (δ_{H} 7.26 ppm, δ_{C} 77.0 ppm) as internal standards. The HSQC experiments were optimized for ¹*J*_{CH} = 145 Hz, and the HMBC experiments for *ⁿJ*_{CH} = 7 or 3.5 Hz. LC-MS data were obtained using an Agilent 1100 equipped with a ThermoFinnigan LCQ by ESI (positive mode). HRMS data were obtained using an Agilent LC-TOF mass spectrometer equipped with an ESI/APCI multimode ion source detector.

### Example 1: Compound 1 physicochemical data

**Largazole (1):** colorless, amorphous solid; [α]²⁰_{D} +22 (*c* 0.1, MeOH); UV (MeOH) (log ∈) 210 (4.07), 260 (sh) (3.61); IR (film) νₘₐₓ 2924, 2853, 1725, 1694, 1611, 1659, 1641, 1630, 1596, 1512, 1249, 1117, 1067, 1034, 894 cm⁻¹; ¹H NMR, ¹³C NMR, and HMBC data, see Table; HR-ESI/APCI-MS *m*/*z* [M + H]⁺ 623.2397 (calcd for C₂₉H₄₃N₄O₅S₃ 623.2396).

### NMR Spectral Data for Largazole (1) in CDCl₃ (600 MHz)

| C/H no. | δ_{H} (*J* in Hz) | δ_{C}, mult. | HMBC*^{a,b}* |
|---|---|---|---|
| 1 | | 168.9, qC | |
| 2 | 4.61, dd (9.2, 3.3) | 57.7, CH | 1,3,4,5,6 |
| 3 | 2.10, m | 34.2, CH | 1,*^{c}* 2*^{c}* |
| 4 | 0.68, d (7.2) | 18.9, CH₃ | 2, 3, 5 |
| 5 | 0.50, d (7.2) | 16.6, CH₃ | 2, 3, 4 |
| 2*-*NH | 7.15, d (9.2) | | 1, 6*^{c}* |
| 6 | | 173.5, qC | |
| 7 | | 84.4, qC | |
| 8a | 4.04, d (-11.4) | 43.3, CH₂ | 6, 7, 10 |
| 8b | 3.27, d (-11.4) | | 6, 7, 9 |
| 9 | 1.87, br s | 24.2, CH₃ | 6, 7, 8 |
| 10 | | 164.6, qC | |
| 11 | | 147.4, qC | |
| 12 | 7.76, s | 124.2, CH | 10,*^{c}* 11, 13 |
| 13 | | 167.9, qC | |
| 14a | 5.29, dd (-17.4, 9.6) | 41.1, CH | 13, 15 |
| 14b | 4.27, dd (-17.4, 2.5) | | 13, 15 |
| 14-NH | 6.45, dd (9.6, 2.5) | | 15*^{c}* |
| 15 | | 169.4, qC | |
| 16a | 2.86, dd (-16.5, 10.5) | 40.5, CH₂ | 15, 17, 18 |
| 16b | 2.68, dd (-16.5, 1.8) | | 15 |
| 17 | 5.66, ddd (10.5, 7.2, 1.8) | 72.0, CH | |
| 18 | 5.51, dd (15.6, 7.2) | 128.4, CH | 17, 20 |
| 19 | 5.82, dt (15.6, 7.2) | 132.7, CH | 17, 20 |
| 20 | 2.31, br q (7.2) (2H) | 32.3, CH₂ | 18, 19, 21 |
| 21 | 2.90, t (7.2) (2H) | 27.9, CH₂ | 19, 20, 22 |
| 22 | | 199.4, qC | |
| 23 | 2.52, t (7.5) (2H) | 44.1, CH₂ | 22, 24, 25 |
| 24 | 1.64, m (2H) | 25.6, CH₂ | 22, 23, 25/26 |
| 25 | 1.29, m (2H) | 28.9, CH₂ | 26 |
| 26 | 1.25, m (2H) | 28.9, CH₂ | 25, 27 |
| 27 | 1.26, m (2H) | 31.6, CH₂ | |
| 28 | 1,28, m (2H) | 22.6, CH₂ | |
| 29 | 0.87, br t (6.9) | 14.0, CH₃ | 27, 28 |

| | | | |
|---|---|---|---|
| *^{a}*Protons showing HMBC correlations to the indicated carbon. *^{b}*Optimized for *ⁿJ* = 7 Hz if not indicated otherwise. *^{c}*Optimized for *ⁿJ* = 3.5 Hz. | | | |

### Example 2: Osteoblast differentiation

The anabolic activity of test compounds was performed by staining ALP that is one of osteoblast differentiation biomarkers. As shown in Fig. 1A and B, low doses of test compounds induced ALP expression even in the absence of BMP-2. Up to 50 nM, these compounds did not show significant cytotoxicity (Fig. 1C). Interestingly, Compound 1 strongly induced the activity of RUNX2 that is key regulator of the commitment of mesenchymal stem cells into osteoblast (Fig. 1D). Most importantly, Compound 1 exhibited anabolic bone forming activity *in vivo* bone regeneration model (Fig. 2). Compounds 1 and 8 have the potential to inhibit the osteoclastogenesis.

### Example 3: Bone Regeneration

All rabbits are anesthetized with an intramuscular dose of 0.1 ml/kg Zoletil (Virbac, France). The head is shaved, and the cutaneous surface is disinfected with povidone iod solution prior to the operation. The calvaria bone is exposed through a skin incision of ∼4 cm in length. Four similar circular bicortical defects (3-mm diameter) are made in the parietal bone using a trephine on a slow-speed electric handpiece, applying 0.9% physiologic saline irrigation. Defects are filled with 10 mg of bone graft substitute, MBCP (Biomatlante, France) soaked with 50 µl of PBS or LA-1. Closure of periosteum and subcutaneous tissues is done with lactomer (Syneture), while the skin is relocated with nylon:polyamide (Syneture). Postoperative antibiotics are administered, e.g., Gentamycin. Rabbits are sacrificed at 4 weeks after surgery.

### Example 4: Osteoclast/Osteoblast differentiation

Osteoclast/Osteoblast differentiation protocols are known in the art. Representative examples applicable to evaluating compounds herein include:
Kim JM, Lee SU, Kim YS, Min YK, Kim SH. Baicalein stimulates osteoblast differentiation via coordinating activation of MAP kinases and transcription factors. J Cell Biochem. 2008 Aug 1;104(5):1906-17;
Kim MH, Ryu SY, Bae MA, Choi JS, Min YK, Kim SH. Baicalein inhibits osteoclast differentiation and induces mature osteoclast apoptosis. Food Chem Toxicol. 2008 Nov;46(1 1):3375-82.

### Example 5: Alkaline phosphatase staining and activity assay.

C2C12 cells were plated at 5 × 10³ cells/well in a 96-well plate and incubated for 1 day and then the medium containing 5% FBS and largazole was changed. The medium was changed every 3 days and on the differentiation day 6, cells were washed twice with PBS, fixed with 10% formalin in PBS for 30 sec, rinsed with deionized water, and stained using the Alkaline Phosphatase (ALP) Kit (Sigma) under protection from direct light. Images of stained cells were captured under a microscope equipped with a DP70 digital camera (Olympus Optical, Japan). To measure ALP activity, cells were washed twice with PBS and sonicated in lysis buffer (10 mM of Tris-HCl, pH 7.5, 0.5 mM of MgCl₂, and 0.1% Triton X-100). After centrifugation at 10,000 × g for 20 min at 4°C, ALP activity in the supernatant was measured in triplicate using the LabAssay ALP Kit (Wako Pure Chemicals Industries). Protein concentration was measured using the BCA Protein Assay kit (Pierce). Significance was determined by Student's *t*-test and differences were considered significant when *P* < 0.05.

### Example 6: Evaluation of mRNA expression level.

Primers were designed using an on-line primer design program (Table 1). (S. Rozen, H. J. Skaletsky, Methods Mol. Biol. 2000, 132, 365-386.) Total RNA was isolated using TRIzol reagent (Life Technologies, MD) according to manufacturer's protocol. The concentration and purity of total RNA were calculated by measuring absorbance at 260 and 280 nm. First strand cDNA was synthesized using 2 µg of total RNA and 1 µM of oligo-dT₁₈ primer and Omniscript Reverse Transcriptase (Qiagen, CA). SYBR green-based quantitative PCR was performed using the Stratagene Mx3000P Real-Time PCR system and Brilliant SYBR Green Master Mix (Stratagene, CA) with 3 µl of first-strand cDNA diluted 1:50 and 20 pmole of primers, according to the manufacturer's protocols. The PCR reaction consisted of three segments. The first segment (95°C for 10 min) activated the polymerase; the second segment included 3-step cycling (40 cycles) at 94°C for 40 sec (denaturation), 60°C for 40 sec (annealing), and 72°C for 1 min (extension); the third segment was performed to generate PCR product temperature dissociation curves ('melting curves') at 95°C for 1 min, 55°C for 30 sec, 95°C for 30 sec. All reactions were run in triplicate, and data were analyzed by the 2^{-ΔΔCT} method (K. J. Livak, T. D. Schmittgen, Methods 2001, 25, 402-408.). GAPDH was used as the control gene. Significance was determined by Student's t-test with GAPDH-normalized 2^{-ΔΔCT} values. Differences were considered significant when *P* < 0.05.

The effect of largazole on the mRNA expression levels of BMPs was evaluated. As expected, largazole significantly induced the expressions of BMP-2, 4, 6, 7, and 9, confirming that the osteogenic activity of largazole is mediated through the increase of the expression and/or activity of Runx2 and BMPs. Therefore, anabolic agents such as largazole that stimulate BMP expression or the BMP-signaling pathway would be useful for the treatment of osteoblast-related diseases by bone formation or regeneration. Thus, the osteogenic activity of largazole can result (at least in part) from its potential to increase the expression/activity of BMPs.

**Table 1. RT-PCR Primers.**

| Target Gene | Forward Primer (5'-3') | Reverse Primer (5'-3') |
|---|---|---|
| *ALP* | GCTGATCATTCCCACGTTTT | CTGGGCCTGGTAGTTGTTGT |
| *OPN* | CGATGATGATGACGATGGAG | TGGCATCAGGATACTGTTCATC |
| *RUNX2* | GCCGGGAATGATGAGAACTA | GGACCGTCCACTGTCACTT |
| *BMP-2* | GCTCCACAAACGAGAAAAGC | AGCAAGGGGAAAAGGACACT |
| *BMP-4* | CCTGGTAACCGAATGCTGAT | AGCCGGTAAAGATCCCTCAT |
| *BMP-6* | TTCTTCAAGGTGAGCGAGGT | TAGTTGGCAGCGTAGCCTTT |
| *BMP-7* | CGATACCACCATCGGGAGTTC | AAGGTCTCGTTGTCAAATCGC |
| *BMP-9* | CAGAACTGGGAACAAGCATCC | GCCGCTGAGGTTTAGGCTG |
| *GAPDH* | AACTTTGGCATTGTGGAAGG | ACACATTGGGGGTAGGAACA |

**Table 2. Effect of largazole on the mRNA induction of osteoblastogenesis markers and BMPs in C2C12 cells. The mRNA levels were evaluated by quantitative real-time PCR in cells (1 × 10⁶ cells/60-mm plate) treated with largazole for 6 days. Fold changes relative to the control are presented as mean ± standard deviation. rhBMP-2 (300 ng/ml) was used as the reference of osteoblastogenesis inducer.**

| Largazole (nM) | Target Genes | | | | | | | |
|---|---|---|---|---|---|---|---|---|
| | *ALP* | *OPN* | *R UNX2* | *BMP-2* | *BMP-4* | *BMP-6* | *BMP-7* | *BMP-9* |
| 0 | 1.00 ± 0.13 | 1.00 ± 0.04 | 1.00 ± 0.07 | 1.00 ± 0.01 | 1.07 ± 0.51 | 1.01 ± 0.18 | 1.00 ± 0.08 | 1.01 ± 0.18 |
| 1 | 1.85 ± 0.06^{a} | 1.33 ± 0.19 | 2.47 ± 0.39^{b} | 2.15 ± 0.42^{b} | 0.91 ± 0.22 | 1.69 ± 0.12^{b} | 5.05 ± 0.31^{c} | 5.18 ± 0.74^{c} |
| 50 | 324.04 ± 1.59^{c} | 2.28 ± 0.12^{b} | 3.66 ± 1.19^{a} | 6.29 ± 0.98^{c} | 2.38 ± 0.91^{a} | 7.15 ± 1.33^{b} | 17.12 ± 2.59^{c} | 12.14 ± 3.17^{b} |
| 100 | 263.33 ± 11.61^{b} | 0.83 ± 0.04^{a} | 2.06 ± 0.00^{c} | 6.34 ± 1.16^{b} | 3.48 ± 1.63 | 8.37 ± 0.88^{c} | 10.72 ± 1.90^{c} | 10.18 ± 2.22^{b} |
| rhBMP-2 | 11.12± 0.00^{c} | 2.35 ± 0.41^{a} | 2.18± 0.48^{a} | 1.45 ± 0.27^{a} | 1.04 ± 0.37 | 1.42 ± 0.35 | 2.85 ± 0.67^{b} | 2.00 ± 0.59^{a} |

| | | | | | | | | |
|---|---|---|---|---|---|---|---|---|
| *^{a}P* < 0.05; *^{b}P* < 0.01; *^{c}P* < 0.001*.* | | | | | | | | |

### Example 7: Runx2 luciferase reporter assay.

To measure Runx2 activity, p6×osteoblast-specific cis-acting element (OSE) 2-luc reporter vector was transiently transfected into C2C12 cells that were seeded in a 96-well plate at 5 × 10³ cells/well as described previously (Kim et al., J. Cell Biochem. 2004, 9, 1239-1247). After 24 h, medium was replaced with DMEM containing 5% FBS with or without tanshinone IIA and/or BMP-2. After 24 h, the cells were lysed, and luciferase activity was measured using luciferase reporter assay system (Promega) and the Wallac EnVision microplate reader.

Since Runx2 has been shown to be critical in osteogenesis and regulates the expression of bone-specific genes such as ALP and OPN, [J. Cell Biochem. 2003, 88, 446-454] we examined the effect of largazole on the activation and mRNA expression of Runx2 using the p6×osteoblast-specific *cis*-acting element 2-luciferase reporter *[*J. Cell Biochem. 2004, 9, 1239-1247] and real-time PCR, respectively. Relative to the DMSO control, largazole at 50 nM significantly increased the activity and mRNA level of Runx2 by 29-fold and 3-fold, respectively (see the Supporting Information for details). Since HDACs have been identified as co-repressor proteins to affect Runx2 activity, [J. Cell Biochem. 2006, 98, 54-64] these results suggested that inhibition of HDACs by largazole increases Runx2 activation, potentiates osteoblast differentiation, and increases bone formation.

### Example 8: In vivo murine calvarial bone formation assay.

*In vivo* bone-forming activity of largazole was evaluated using lyophilized collagen sponges as described previously. (H. Ha, J. H. Lee, H. N. Kim, H. M. Kim, H. B. Kwak, S. Lee, H. H. Kim, Z. H. Lee, J. Immunol. 2006, 176, 111-117.) Briefly, collagen sponges loaded with 5 µl of vehicle or largazole were implanted over the calvarial bones of mice (n = 5 per group). Three weeks after drug implantation, the calvarias were harvested, fixed in 4% paraformaldehyde, decalcified in 12% EDTA, embedded in paraffin, and sectioned. Sections were deparaffinized through graded xylene washes, dehydrated in a graded series of ethanol washes, and stained with hematoxylin and eosin (H&E).

In the mouse calvarial bone formation assay, when collagen sponges soaked with largazole were implanted in calvarial bones, largazole induced woven bone formation over the periosteum of the calvarial bones. The woven bone formation at the lower concentration (50 µM) was more significant than that at the higher concentration (100 µM). This data suggested that largazole may show the biphasic effect which has been observed with other osteogenic compounds.

### Example 9: Induction of multinucleated osteoclasts.

RAW264.7 cells were purchased from the ATCC and maintained in DMEM supplemented with 10% FBS, 100 U/ml of penicillin, and 100 µg/ml streptomycin with a change of medium every 3 days in humidified atmosphere of 5% CO₂ in air at 37°C. For the osteoclast differentiation, RAW264.7 cells were suspended in α-minimal essential medium (α-MEM) supplemented with 10% FBS and 100 ng/ml RANKL (R&D Systems Inc., MN) and plated in a 96-well plate at 1 × 10³ cells/well. The next day, cells were treated with largazole and then after an additional 3 days, multinucleated osteoclasts were observed.

### Example 10: Tartrate-resistant acid phosphatase (TRAP) staining and activity.

Multinucleated osteoclasts were fixed with 10% formalin for 10 min and ethanol/acetone (1:1) for 1 min, and then stained by Leukocyte Acid Phosphatase Kit 387-A (Sigma, MO). The images of TRAP-positive multinucleated cells were captured under a microscope with DP Controller (Olympus Optical, Japan). For measuring TRAP activity, multinucleated osteoclasts were fixed with 10% formalin for 10 min and 95% ethanol for 1 min, and then 100 µl of citrate buffer (50 mM, pH 4.6) containing 10 mM sodium tartrate, and 5 mM *p*-nitrophenylphosphate (Sigma) was added to the dried cells. After incubation for 1 h, the enzyme reaction mixtures in the wells were transferred into new plates containing an equal volume of 0.1 N NaOH. Absorbance was measured at 410 nm, and TRAP activity was presented as % of control. The experiment was performed in triplicate.

### References

(1) (a) Koehn, F. E.; Carter, G. T. Nat. Rev. Drug Discov. 2005, 4, 206-220. (b) Paterson, I.; Anderson, E. A. Science 2005, 310, 451-453.
(2) Fenical, W.; Jensen, P. R. Nat. Chem. Biol. 2006, 2, 666-673.
(3) Gerwick, W. H.; Tan, L. T.; Sitachitta, N. Alkaloids Chem. Biol. 2001, 57, 75-184.
(4) Luesch, H.; Moore, R. E.; Paul, V. J.; Mooberry, S. L.; Corbett, T. H. J. Nat. Prod. 2001, 64, 907-910.
(5) (a) Gerwick, W. H.; Proteau, P. J.; Nagle, D. G.; Hamel, E.; Blokhin, A.; Slate, D. L. J. Org. Chem. 1994, 59, 1243-1245. (b) Verdier-Pinard, P.; Lai, J.-Y.; Yoo, H.-D.; Yu, J.; Marquez, B.; Nagle, D. G.; Nambu, M.; White, J. D.; Falck, J. R.; Gerwick, W. H.; Day, B. W.; Hamel, E. Mol. Pharmacol. 1998, 53, 62-76.
(6) (a) Luesch, H.; Yoshida, W. Y.; Moore, R. E.; Paul, V. J.; Corbett, T. H. J. Am. Chem. Soc. 2001, 123, 5418-5423. (b) Luesch, H.; Chanda, S. K.; Raya, M. R.; DeJesus, P. D.; Orth, A. P.; Walker, J. R.; Izpisúa Belmonte, J. C.; Schultz, P. G. Nat. Chem. Biol. 2006, 2, 158-167.
(7) (a) Carmeli, S.; Moore, R. E.; Patterson, G. M. L. Tetrahedron Lett. 1991, 32, 2593-2596. (b) Pattenden, G.; Thom, S. M. J. Chem. Soc. Perkin Trans. I 1993, 1629-1636. (c) Boyce, R. J.; Pattenden, G. Tetrahedron 1995, 51, 7313-7320.
(8) Carmeli, S.; Moore, R. E.; Patterson, G. M. L. J. Am. Chem. Soc. 1990, 112, 8195-8197.
(9) Horton, P.; Inman, W. D.; Crews, P. J. Nat. Prod. 1990, 53, 143-151.
(10) (a) Roller, P.; Au, K.; Moore, R. E. Chem. Commun. 1971, 503-504. (b) Sata, N.; Abinsay, H.; Yoshida, W. Y.; Horgen, F. D.; Sitachitta, N.; Kelly, M.; Scheuer, P. J. J. Nat. Prod. 2005, 68, 1400-1403.
(11) (a) Perez Baz, J.; Cañedo, L. M.; Fernández, Puentes, J. L.; Silva Elipe, M. V. J. Antibiot. (Tokyo) 1997, 50, 738-741. (b) Boger, D. G.; Ichikawa, S. J. Am. Chem. Soc. 2000, 122, 2956-2957.

## Claims

1. A pharmaceutical composition comprising the compound of formula I: 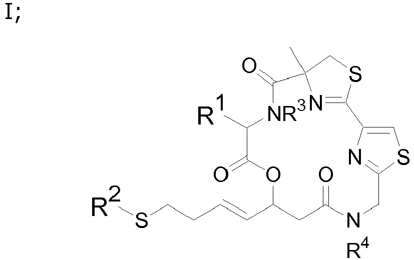 wherein:
each R is independently H, or alkyl;
each R¹ is independently H, or alkyl;
each R² is independently H, alkyl, or C(O)R;
each R³ is independently H, alkyl, C(O)OR, or C(O)NRR;
each R⁴ is independently H, alkyl, C(O)OR, or C(O)NRR;
and pharmaceutically acceptable salts, solvate, or hydrate thereof, an anti-osteoporotic agent, and a pharmaceutically acceptable carrier.

2. The pharmaceutical composition of claim 1, wherein the compound of formula I is any of Compounds 1-8 in table A below:
**Table A**
| Cmpd No. | R¹ | R² | R³ | R⁴ |
|---|---|---|---|---|
| 1 | isopropyl | n-heptylC(O)- | H | H |
| 2 | isopropyl | n-heptylC(O)- | H | Me |
| 3 | isopropyl | Me | H | H |
| 4 | isopropyl | n-heptylC(O)- | H | methylC(O)- |
| 5 | isopentyl | n-heptylC(O)- | H | H |
| 6 | ethyl | n-heptylC(O)- | Me | Me |
| 7 | isopropyl | CH₃C(O)- | H | H |
| 8 | isopropyl | H | H | H |
or pharmaceutically acceptable salt thereof.

3. A kit comprising an effective amount of a compound of formula I: 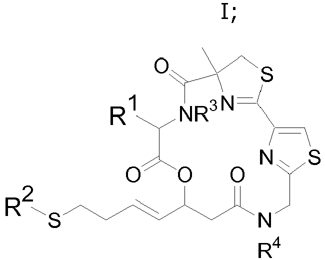 wherein:
each R is independently H, or alkyl;
each R¹ is independently H, or alkyl;
each R² is independently H, alkyl, or C(O)R;
each R³ is independently H, alkyl, C(O)OR, or C(O)NRR;
each R⁴ is independently H, alkyl, C(O)OR, or C(O)NRR;
and pharmaceutically acceptable salts, solvate, or hydrate thereof, in unit dosage form, for use in treating a subject suffering from osteoporosis.

4. A compound of formula I: 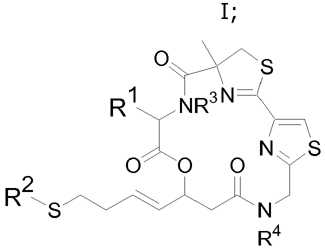 wherein:
each R is independently H, or alkyl;
each R¹ is independently H, or alkyl;
each R² is independently H, alkyl, or C(O)R;
each R³ is independently H, alkyl, C(O)OR, or C(O)NRR;
each R⁴ is independently H, alkyl, C(O)OR, or C(O)NRR;
and pharmaceutically acceptable salts, solvate, or hydrate thereof, or a pharmaceutical composition thereof; for use in treating osteoporosis.

5. The compound for use according to claim 4, wherein the compound has anabolic activity and anti-resorptive activity.

6. The compound for use according to claim 4, wherein the compound of formula I is one of Compounds 1-8 in table A below:
**Table A**
| Cmpd No. | R¹ | R² | R³ | R⁴ |
|---|---|---|---|---|
| 1 | isopropyl | n-heptylC(O)- | H | H |
| 2 | isopropyl | n-heptylC(O)- | H | Me |
| 3 | isopropyl | Me | H | H |
| 4 | isopropyl | n-heptylC(O)- | H | methylC(O)- |
| 5 | isopentyl | n-heptylC(O)- | H | H |
| 6 | ethyl | n-heptylC(O)- | Me | Me |
| 7 | isopropyl | CH₃C(O)- | H | H |
| 8 | isopropyl | H | H | H |

7. The compound for use according to claim 4, wherein the subject is a mammal.

8. The compound for use according to claim 4, wherein the subject is a primate or human.

9. The compound for use according to claim 4, wherein the compound of claim 1 is to be administered in amounts from about 0.005 µg/kg to about 200 mg/kg; from about 0.1 mg/kg to about 200 mg/kg; from about 10 mg/kg to 100 mg/kg; or from about 1.0 pM to about 500 nM.

10. The compound for use according to claim 4, wherein the compound of formula I is to be administered intravenously, intramuscularly, subcutaneously, intracerebroventricularly, orally or topically.

11. The compound for use according to claim 4, wherein the compound of formula I is to be administered alone or in combination with one or more other therapeutics.

12. The compound for use according to claim 11, wherein the additional therapeutic agent is an anti-osteoporotic agent.

13. A compound for use according to claim 4, wherein said compound is a pharmaceutically acceptable salt of any of Compounds 1-8 in table A below:
**Table A**
| Cmpd No. | R¹ | R² | R³ | R⁴ |
|---|---|---|---|---|
| 1 | isopropyl | n-heptylC(O)- | H | H |
| 2 | isopropyl | n-heptylC(O)- | H | Me |
| 3 | isopropyl | Me | H | H |
| 4 | isopropyl | n-heptylC(O)- | H | methylC(O)- |
| 5 | isopentyl | n-heptylC(O)- | H | H |
| 6 | ethyl | n-heptylC(O)- | Me | Me |
| 7 | isopropyl | CH₃C(O)- | H | H |
| 8 | isopropyl | H | H | H |

## Patentansprüche

1. Pharmazeutische Zusammensetzung, umfassend die Verbindung der Formel I: 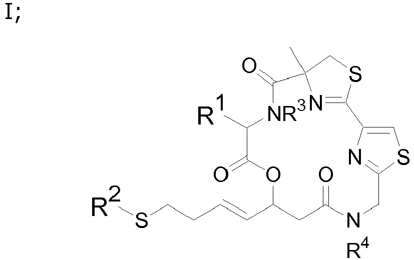 wobei:
jedes R unabhängig H oder Alkyl ist;
jedes R¹ unabhängig H oder Alkyl ist;
jedes R² unabhängig H, Alkyl oder C(O)R ist;
jedes R³ unabhängig H, Alkyl, C(O)OR oder C(O)NRR ist;
jedes R⁴ unabhängig H, Alkyl, C(O)OR oder C(O)NRR ist;
und pharmazeutisch unbedenkliche Salze, ein Solvat oder ein Hydrat davon, einen anti-osteoporotischen Wirkstoff und einen pharmazeutisch unbedenklichen Träger.

2. Pharmazeutische Zusammensetzung nach Anspruch 1, wobei die Verbindung der Formel I irgend eine der Verbindungen 1-8 in Tabelle A unten ist:
**Tabelle A**
| Verbindung Nr. | R¹ | R² | R³ | R⁴ |
|---|---|---|---|---|
| 1 | Isopropyl | n-HeptylC(O)- | H | H |
| 2 | Isopropyl | n-HeptylC(O)- | H | Me |
| 3 | Isopropyl | Me | H | H |
| 4 | Isopropyl | n-HeptylC(O)- | H | MethylC(O)- |
| 5 | Isopropyl | n-HeptylC(O)- | H | H |
| 6 | Ethyl | n-HeptylC(O)- | Me | Me |
| 7 | Isopropyl | CH₃CO(O)- | H | H |
| 8 | Isopropyl | H | H | H |
oder ein pharmazeutisch unbedenkliches Salz davon.

3. Kit umfassend eine wirksame Menge einer Verbindung der Formel I: 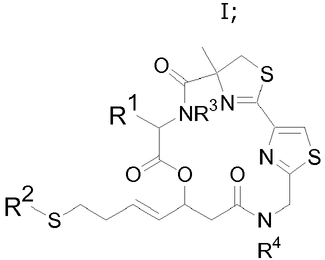 wobei:
jedes R unabhängig H oder Alkyl ist;
jedes R¹ unabhängig H oder Alkyl ist;
jedes R² unabhängig H, Alkyl oder C(O)R ist;
jedes R³ unabhängig H, Alkyl, C(O)OR oder C(O)NRR ist;
jedes R⁴ unabhängig H, Alkyl, C(O)OR oder C(O)NRR ist;
und pharmazeutisch unbedenkliche Salze, ein Solvat oder ein Hydrat davon, in Einheitsdosierungsform, zur Verwendung beim Behandeln eines Subjekts, das an Osteoporose leidet.

4. Verbindung der Formel I: 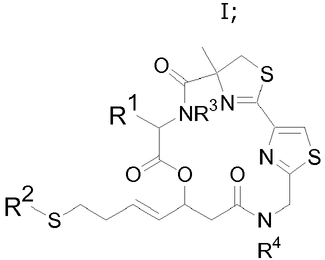 wobei:
jedes R unabhängig H oder Alkyl ist;
jedes R¹ unabhängig H oder Alkyl ist;
jedes R² unabhängig H, Alkyl oder C(O)R ist;
jedes R³ unabhängig H, Alkyl, C(O)OR oder C(O)NRR ist;
jedes R⁴ unabhängig H, Alkyl, C(O)OR oder C(O)NRR ist;
und pharmazeutisch unbedenkliche Salze, ein Solvat oder ein Hydrat davon, oder
eine pharmazeutische Zusammensetzung davon; zur Verwendung beim Behandeln von Osteoporose.

5. Verbindung zur Verwendung nach Anspruch 4, wobei die Verbindung eine anabolische Aktivität und eine antiresorptive Aktivität hat.

6. Verbindung zur Verwendung nach Anspruch 4, wobei die Verbindung der Formel I eine der Verbindungen 1-8 in Tabelle A unten ist:
**Tabelle A**
| Verbindung Nr. | R¹ | R² | R³ | R⁴ |
|---|---|---|---|---|
| 1 | Isopropyl | n-HeptylC(O)- | H | H |
| 2 | Isopropyl | n-HeptylC(O)- | H | Me |
| 3 | Isopropyl | Me | H | H |
| 4 | Isopropyl | n-HeptylC(O)- | H | MethylC(O)- |
| 5 | Isopropyl | n-HeptylC(O)- | H | H |
| 6 | Ethyl | n-HeptylC(O)- | Me | Me |
| 7 | Isopropyl | CH₃CO(O)- | H | H |
| 8 | Isopropyl | H | H | H |

7. Verbindung zur Verwendung nach Anspruch 4, wobei es sich bei dem Subjekt um ein Säugetier handelt.

8. Verbindung zur Verwendung nach Anspruch 4, wobei es sich bei dem Subjekt um einen Primaten oder einen Menschen handelt.

9. Verbindung zur Verwendung nach Anspruch 4, wobei die Verbindung von Anspruch 1 in den folgenden Mengen zu verabreichen ist: von etwa 0,005 µg/kg bis etwa 200 mg/kg; von etwa 0,1 mg/kg bis etwa 200 mg/kg; von etwa 10 mg/kg bis 100 mg/kg; oder von etwa 1,0 pM bis etwa 500 nM.

10. Verbindung zur Verwendung nach Anspruch 4, wobei die Verbindung der Formel I intravenös, intramuskulär, subkutan, intracerebroventrikulär, oral oder topisch zu verabreichen ist.

11. Verbindung zur Verwendung nach Anspruch 4, wobei die Verbindung der Formel I allein oder in Kombination mit einem oder mehreren Therapeutika zu verabreichen ist.

12. Verbindung zur Verwendung nach Anspruch 11, wobei das zusätzliche Therapeutikum ein anti-osteoporotischer Wirkstoff ist.

13. Verbindung zur Verwendung nach Anspruch 4, wobei die Verbindung ein pharmazeutisch unbedenkliches Salz von jeder der Verbindungen 1-8 in Tabelle A unten ist:
**Tabelle A**
| Verbindung Nr. | R¹ | R² | R³ | R⁴ |
|---|---|---|---|---|
| 1 | Isopropyl | n-HeptylC(O)- | H | H |
| 2 | Isopropyl | n-HeptylC(O)- | H | Me |
| 3 | Isopropyl | Me | H | H |
| 4 | Isopropyl | n-HeptylC(O)- | H | MethylC(O)- |
| 5 | Isopropyl | n-HeptylC(O)- | H | H |
| 6 | Ethyl | n-HeptylC(O)- | Me | Me |
| 7 | Isopropyl | CH₃CO(O)- | H | H |
| 8 | Isopropyl | H | H | H |

## Revendications

1. Composition pharmaceutique comprenant le composé de formule I : 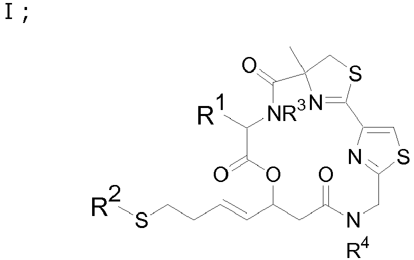 dans laquelle :
chaque groupe R représente indépendamment un atome H ou un groupe alkyle ;
chaque groupe R¹ représente indépendamment un atome H ou un groupe alkyle ;
chaque groupe R² représente indépendamment un atome H, un groupe alkyle ou C(O)R ;
chaque groupe R³ représente indépendamment un atome H, un groupe alkyle, C(O)OR ou C(O)NRR ;
chaque groupe R⁴ représente indépendamment un atome H, un groupe alkyle, C(O)OR ou C(O)NRR ;
et des sels, un solvate ou un hydrate pharmaceutiquement acceptables de celui-ci, un agent anti-ostéoporotique et un excipient pharmaceutiquement acceptable.

2. Composition pharmaceutique selon la revendication 1, ledit composé de formule I étant l'un quelconque des composés 1 à 8 dans le tableau A ci-dessous :
**Tableau A**
| Composé n° | R¹ | R² | R³ | R⁴ |
|---|---|---|---|---|
| 1 | isopropyle | n-heptylC(O)- | H | H |
| 2 | isopropyle | n-heptylC(O)- | H | Me |
| 3 | isopropyle | Me | H | H |
| 4 | isopropyle | n-heptylC(O)- | H | méthylC(O)- |
| 5 | isopropyle | n-heptylC(O)- | H | H |
| 6 | éthyle | n-heptylC(O)- | Me | Me |
| 7 | isopropyle | CH₃CO(O)- | H | H |
| 8 | isopropyle | H | H | H |
ou un sel pharmaceutiquement acceptable de ceux-ci.

3. Kit comprenant une quantité efficace d'un composé de formule I : 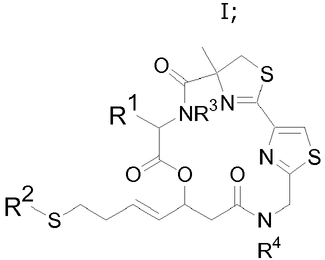 dans laquelle :
chaque groupe R représente indépendamment un atome H ou un groupe alkyle ;
chaque groupe R¹ représente indépendamment un atome H ou un groupe alkyle ;
chaque groupe R² représente indépendamment un atome H, un groupe alkyle ou C(O)R ;
chaque groupe R³ représente indépendamment un atome H, un groupe alkyle, C(O)OR ou C(O)NRR ;
chaque groupe R⁴ représente indépendamment un atome H, un groupe alkyle, C(O)OR ou C(O)NRR ;
et des sels, un solvate ou un hydrate pharmaceutiquement acceptables de celui-ci, en forme posologique unitaire, pour utilisation dans le traitement d'un sujet souffrant d'ostéoporose.

4. Composé de formule I : 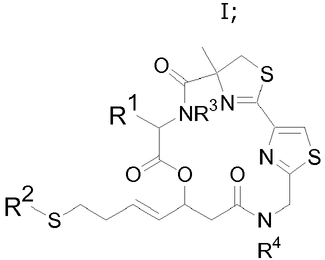 dans laquelle :
chaque groupe R représente indépendamment un atome H ou un groupe alkyle ;
chaque groupe R¹ représente indépendamment un atome H ou un groupe alkyle ;
chaque groupe R² représente indépendamment un atome H, un groupe alkyle ou C(O)R ;
chaque groupe R³ représente indépendamment un atome H, un groupe alkyle, C(O)OR ou C(O)NRR ;
chaque groupe R⁴ représente indépendamment un atome H, un groupe alkyle, C(O)OR ou C(O)NRR ;
et sels, solvate ou hydrate pharmaceutiquement acceptables de celui-ci, ou composition pharmaceutique de ceux-ci ; pour utilisation dans le traitement de l'ostéoporose.

5. Composé pour utilisation selon la revendication 4, ledit composé présentant une activité anabolique et une activité anti-résorption.

6. Composé pour utilisation selon la revendication 4, ledit composé de formule I étant l'un des composés 1 à 8 dans le tableau A ci-dessous :
**Tableau A**
| Composé n° | R¹ | R² | R³ | R⁴ |
|---|---|---|---|---|
| 1 | isopropyle | n-heptylC(O)- | H | H |
| 2 | isopropyle | n-heptylC(O)- | H | Me |
| 3 | isopropyle | Me | H | H |
| 4 | isopropyle | n-heptylC(O)- | H | méthylC(O)- |
| 5 | isopropyle | n-heptylC(O)- | H | H |
| 6 | éthyle | n-heptylC(O)- | Me | Me |
| 7 | isopropyle | CH₃CO(O)- | H | H |
| 8 | isopropyle | H | H | H |

7. Composé pour utilisation selon la revendication 4, le sujet étant un mammifère.

8. Composé pour utilisation selon la revendication 4, le sujet étant un primate ou un humain.

9. Composé pour utilisation selon la revendication 4, ledit composé selon la revendication 1 étant destiné à être administré en quantités d'environ 0,005 µg/kg à environ 200 mg/kg ; d'environ 0,1 mg/kg à environ 200 mg/kg ; d'environ 10 mg/kg à 100 mg/kg ; ou d'environ 1,0 pM à environ 500 nM.

10. Composé pour utilisation selon la revendication 4, ledit composé de formule I étant destiné à être administré par voie intraveineuse, intramusculaire, sous-cutanée, intracérébroventriculaire, orale ou topique.

11. Composé pour utilisation selon la revendication 4, ledit composé de formule I étant destiné à être administré seul ou en combinaison avec un ou plusieurs autres agents thérapeutiques.

12. Composé pour utilisation selon la revendication 11, ledit agent thérapeutique supplémentaire étant un agent anti-ostéoporotique.

13. Composé pour utilisation selon la revendication 4, ledit composé étant un sel pharmaceutiquement acceptable de l'un quelconque des composés 1 à 8 dans le tableau A ci-dessous :
**Tableau A**
| Composé n° | R¹ | R² | R³ | R⁴ |
|---|---|---|---|---|
| 1 | isopropyle | n-heptylC(O)- | H | H |
| 2 | isopropyle | n-heptylC(O)- | H | Me |
| 3 | isopropyle | Me | H | H |
| 4 | isopropyle | n-heptylC(O)- | H | méthylC(O)- |
| 5 | isopropyle | n-heptylC(O)- | H | H |
| 6 | éthyle | n-heptylC(O)- | Me | Me |
| 7 | isopropyle | CH₃CO(O)- | H | H |
| 8 | isopropyle | H | H | H |
